(19) **Europäisches Patentamt European Patent Office Office européen des brevets**

(11) **EP 2 959 911 B1**

(12) **EUROPEAN PATENT SPECIFICATION**

(45) Date of publication and mention
of the grant of the patent:
**31.07.2019 Bulletin 2019/31**

(21) Application number: **14810943.2**

(22) Date of filing: **13.05.2014**

(51) Int Cl.:
*A61K 36/634* *(2006.01)*    *A61K 31/51* *(2006.01)*
*A61K 31/375* *(2006.01)*    *A61K 9/107* *(2006.01)*
*A61K 47/34* *(2017.01)*    *A61K 47/14* *(2017.01)*
*A61K 47/10* *(2017.01)*    *A61P 31/00* *(2006.01)*
*A61P 11/00* *(2006.01)*    *A61K 36/233* *(2006.01)*

(86) International application number:
**PCT/CN2014/077352**

(87) International publication number:
**WO 2014/198173 (18.12.2014 Gazette 2014/51)**

(54) **COMPOUND FRUCTUS FORSYTHIAE-RADIX BUPLEURI OIL NANOEMULSION PREPARATION**

VERBINDUNG ZUR HERSTELLUNG EINER
FRUCTUS-FORSYTHIAE-RADIX-BUPLEURI-ÖLNANOEMULSION

PRÉPARATION D'UNE NANOÉMULSION D'UN COMPOSÉ D'HUILE DE FRUCTUS
FORSYTHIAE-RADIX BUPLEURI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2013 CN 201310230521**

(43) Date of publication of application:
**30.12.2015 Bulletin 2015/53**

(73) Proprietor: **Zhang, Xiaoyan
Tianjin 300161 (CN)**

(72) Inventor: **Zhang, Xiaoyan
Tianjin 300161 (CN)**

(74) Representative: **Hanna Moore + Curley
Garryard House
25/26 Earlsfort Terrace
Dublin 2, D02 PX51 (IE)**

(56) References cited:
**CN-A- 102 166 234    CN-A- 103 301 204**

- **DATABASE WPI Week 200918 Thomson
Scientific, London, GB; AN 2009-B47955
XP002751211, & CN 101 322 749 A (GUANGXI
YUANANTANG MEDICINE CO LTD) 17 December
2008 (2008-12-17)**

- **DATABASE WPI Week 200944 Thomson
Scientific, London, GB; AN 2009-J84808
XP002751212, & CN 101 433 658 A (TAIAN
SHANNONGDA PHARM CO LTD) 20 May 2009
(2009-05-20)**
- **DATABASE WPI Week 201129 Thomson
Scientific, London, GB; AN 2011-B47809
XP002751213, & CN 101 933 976 A (UNIV
NORTHWESTERN) 5 January 2011 (2011-01-05)**
- **DATABASE WPI Week 201279 Thomson
Scientific, London, GB; AN 2012-N57118
XP002751214, & CN 102 600 240 A (UNIV
NORTHWEST A & F) 25 July 2012 (2012-07-25)**
- **ZHANG WENJUN ET AL: "Technology for
improving the bioavailability of small molecules
extracted from traditional Chinese medicines",
EXPERT OPINION ON DRUG DELIVERY,
INFORMA HEALTHCARE, GB, vol. 6, no. 11, 1
November 2009 (2009-11-01), pages 1247-1259,
XP009172340, ISSN: 1742-5247, DOI:
10.1517/17425240903206963**
- **DANG, QING ET AL.: 'Preparation and in vitro
Evaluation for Nanoemulsion of Forsythia
Suspense Oil' CHINESE JOURNAL OF
PHARMACEUTICAL BIOTECHNOLOGY vol. 18,
no. 2, 31 December 2011, pages 157 - 158,
XP055228160**
- **CHEN, XINQIAN ET AL.: 'Vitamin C and Vitamin
B' NEW MATERIA MEDICA vol. 30, no. 15, 31
March 2003, page 628, 630, XP008178113**

EP 2 959 911 B1

Remarks:
The file contains technical information submitted after the application was filed and not included in this specification

Remarks:
The file contains technical information submitted after the application was filed and not included in this specification

2

**Description**

**Technical Field of the Invention**

The present invention, pertaining to the medical field, relates to a compound Forsythia suspensa-Radix bupleuri oil nanoemulsion preparation, and particularly relates to a transparent and stable compound Forsythia suspensa-Radix bupleuri oil nanoemulsion preparation.

**Background of the Invention**

Forsythia suspensa is a Chinese medicine for clearing away heat and removing toxic substances with antibacterial, heart-tonifying, diuresis-promoting, and vomiting-stopping pharmacological effects, etc., it mainly treats early pyreticosis, anemopyretic cold, fever, vexation, laryngopharynx swelling and pain, and acute nephritis, etc. The main components of Forsythia suspensa volatile oil are B-pinene, A-pinene, etc. The Forsythia suspensa volatile oil has antibacterial and anti-virus effects, and has obvious effect for inhibiting staphylococcus aureus, diplococcus pneumoniae, and Candida albicans. The volatile essential oil (from yellowish-brown to brown) of Radix bupleuri oil has antipyretic, YANG-invigorating, and liver-soothing effects, which is used for treating fever due to common cold, alternate attack of chill and fever, malaria, stagnation of QI due to depression of the liver, and distending pain in the chest and hypochondrium. At present, the mixture of compound Forsythia suspense oil and Radix bupleuri oil does not have a nanoemulsion preparation. The Forsythia suspense oil and Radix bupleuri oil have good curative efficacy with small toxic side effect, however, they are insoluble in water and they are volatile, and have low stability and low bioavailability, thus the therapeutic effect is affected. Vitamin C, also called L-ascorbic acid, is water-soluble vitamin. The vitamin C in food is absorbed by upper small intestine of human body. Normally, most vitamin C is decomposed in vivo into oxalic acid by metabolism or forms ascorbic acid-2-sulfuric acid by combining with sulfuric acid and is excreted in the urine; the other portion may directly be excreted in vitro in the urine. Vitamin $B_1$ is mainly for the prevention and treatment of vitamin $B_1$ deficiency, such as "beriberi", peripheral neuritis and dyspepsia. Supplement of vitamin $B_1$ is needed under the following circumstances: during pregnancy or lactation, hyperthyroidism, burns, long-term chronic infection, heavy physical labor, malabsorption syndrome accompanied by liver and gall diseases, disease of the small intestine system, and after gastrectomy.

**Summary of the Invention**

In view of the above problems and defects in the prior art, an object of the present invention is to provide a compound Forsythia suspensa-Radix bupleuri oil nanoemulsion preparation which has uniform and accurate content, stable quality, and high bioavailability, and is easy to prepare.
The technical solution for realizing the above object is a compound Forsythia suspensa-Radix bupleuri oil nanoemulsion preparation, comprising raw materials with the following percentages by weight:
12.00%-28.00% of a surfactant, 15.00%-30.00% of a co-surfactant, 3.00%-8.00% of oil, 0.10%-5.00% of Forsythia suspensa oil, 0.10%-5.00% of Radix bupleuri oil, 0.01%-0.05% of vitamin C, 0.01 %-0.05% of vitamin $B_1$ and 20.00%-68.40% of distilled water, and the total percentages by weight of all the raw materials is 100% and further characterized in that the preparation is a spray in the air preparation and has antipyretic, antibacterial and virus-inhibiting effects.
A preferred formulation of the compound Forsythia suspensa-Radix bupleuri oil nanoemulsion preparation comprises raw materials with the following percentages by weight:
16.00%-28.00% of a surfactant, 16.00%-30.00% of a co-surfactant, oil 4.00%-8.00% of oil, 1.00%-5.00% of Forsythia suspensa oil, 1.00%-5.00% of Radix bupleuri oil, 0.01%-0.05% of vitamin C, 0.01%-0.05% of vitamin $B_1$ and 20.00%-60.00% of distilled water, and the total percentages by weight of all the raw materials is 100% and further characterized in that the preparation is a spray in the air preparation and has antipyretic, antibacterial and virus-inhibiting effects.
The best formulation of the compound Forsythia suspensa-Radix bupleuri oil nanoemulsion preparation comprises raw materials with the following percentages by weight:
A compound Forsythia suspensa-Radix bupleuri oil nanoemulsion preparation, characterized in that, it is prepared from raw materials with the following percentages by weight: 24.00% of a surfactant phase, 30.00% of a co-surfactant, 7.00% of oil, 3.00% of Forsythia suspensa oil, 3.00% of Radix bupleuri oil, 0.01% of vitamin C, 0.01% of vitamin $B_1$ and 32.98% of distilled water, and the total percentages by weight of all the raw materials is 100%; and further characterized in that the preparation is a spray in the air preparation and has antipyretic, antibacterial and virus-inhibiting effects.
The surfactant is Tween-80;
The co-surfactant is ethanol;
The oil is ethyl acetate.
A nonionic surfactant with no toxin and good biocompatibility is selected by the present invention for the selection of surfactants. The nonionic surfactant is relatively stable in solution, is less susceptible to strong electrolyte or inorganic salts or acid-bases, and it has good compatibility with other surfactants, and hemolysis is small. Theoretically, HLB value of the surfactant needs to be between 8 and 18 for the preparation of O/W type nanoemulsion, but a liquid nonionic surfactant with HLB between 10 and 15 is selected by the invention in consideration of the simplicity of preparation process, i.e. easy formation of nanoemulsion and stability of the prepared nanoemulsion. The surfactant is Tween-80.

A co-surfactant with small irritation mainly including ethanol, is selected by the invention in order to adjust the hydrophilic-lipophilic balance (HLB) value of the surfactant, can further reduce interfacial tension, enhance flexibility and rigidity of membrane, insert the surfactant into the interfacial film, promote the formation of the membrane with very small curvature radius and enlarge the formation region of the nanoemulsion.

Ethyl acetate is selected as oil by the invention for use according to the principle of "when HLB of the surfactant needed by the emulsified oil phase is close to that of the surfactant phase, the resulting emulsion is stable". The oil is liquid at room temperature.

Preparation method of the compound Forsythia suspensa-Radix bupleuri oil nanoemulsion preparation of the present invention specifically comprises the following steps:

(1) preparation of the surfactant phase: weigh the surfactant according to the formulation ratio, and compound with the co-surfactant according to the ratio, calculate HLB value of the system and stir evenly and fully. HLB values of common emulsifiers can be found in some chemical manuals, such as "Handbook of Chemical Products" published by the Chemical Industry Press. Since the hydrophile-lipophile balance (HLB) value of the surfactant is adductive, HLB value of the surfactant can be obtained by quality averaging method. For example, after mixing surfactant A and surfactant B, the $HLB_{AB}$ of the mixed surfactant is

$$HLB_{AB}=(W_{A}HLB_{A}+W_{B}HLB_{B})/(W_{A}+W_{B})$$

In formula $W_A$, $W_B$ - - weight of the surfactant A and the surfactant B of the mixture;

$HLB_A$, $HLB_B$ - HLB value of the surfactant A and the surfactant B.

(2) preparation of oil phase: select oil according to the HLB value of the surfactant phase, adjust ratio of the oil to make the HLB value needed thereby to be close to that of the surfactant phase.

(3) change ratio of the surfactant phase and the oil phase according to the regularity of 9:1 to 1:9, add formulation ratio of Forsythia suspensa oil therein, stir to fully dissolve it, slowly add distilled water at 20 DEG C to 25 DEG C and fully stir until forming a faint yellow O/W nanoemulsion which is clear and transparent, has a small viscosity and has liquidity.

The invention has an appearance of faint yellow transparent liquid with excellent stability: durational stability refers to the extent to which the appearance of nanoemulsion varies with time when the nanoemulsion is stored under natural varying condition at room temperature. The durational stability is proved preferable as the Forsythia suspensa oil nanoemulsion preparation is durably transparent without finding turbidity or precipitate, which is an important criterion for evaluating nanoemulsion oral liquid. Place the Forsythia suspensa oil nanoemulsion oral liquid in a tube, seal, centrifuge at a rotating speed of 12000r/min for 20 minutes, the oral liquid is not delaminated and is still clear and transparent.

The compound Forsythia suspensa-Radix bupleuri oil nanoemulsion preparation of the present invention has antipyretic, antibacterial and virus-inhibiting effects, and has obvious effect for inhibiting staphylococcus aureus, diplococcus pneumoniae, and Candida albicans. Clinically, it can be used for treating heat cold, upper respiratory tract infection, etc. Usage: take 50 ml three times a day for oral administration; apply externally below the eyes and nose; spray into the nose; spray in the air.

**Compared with the prior art, the compound Forsythia suspensa-Radix bupleuri oil nanoemulsion preparation of the present invention has the following advantages:**

1) The thermodynamic stability is high. Operation for the preparation is relatively simple, and there is no phase separation and no precipitation, and the storage stability is improved;

2) The transmittance is good, any heterogeneity or presence of precipitate is easy to be detected, and the sensory quality is improved;

3) It has preferable solubilization, which can effectively improve the solubility of poorly water soluble drugs;

4) It can improve the solubility of Forsythia suspensa oil and Radix bupleuri oil, delay the recession time of Forsythia suspensa oil, thereby improving the bioavailability of Forsythia suspensa oil;

5) The process is simple and is suitable for large scale production.

**Detailed Description of the Invention**

Preparation method of the compound Forsythia suspensa-Radix bupleuri oil nanoemulsion preparation of the present invention will be further illustrated below with Examples given by the inventor.

**Example 1**

Accurately weigh 28.0 g of Tween-80, 28.0 g of ethanol and 8.0 g of ethyl acetate, place them into a beaker, manually stir to fully and homogeneously mix the liquid at room temperature of 20 DEG C, directly add 0.1 g of Forsythia suspensa oil, 0.1 g of Radix bupleuri oil, 0.05 g of vitamin C and 0.05 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 2**

Accurately weigh 12.0 g of Tween-80, 15.0 g of ethanol and 3.0 g of ethyl acetate, place them into a beaker, manually stir to fully and homogeneously mix the liquid at room temperature of 20 DEG C, directly add 1.0 g of Forsythia suspensa oil, 1.0 g of Radix bupleuri oil, 0.03 g of vitamin C and 0.03 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.1 g.

**Example 3**

Accurately weigh 16.0 g of Tween-80, 20.0 g of ethanol and 4.0 g of ethyl acetate, place them into a beaker, manually stir to fully and homogeneously mix the liquid at room temperature of 20 DEG C, directly add 1.5 g of Forsythia suspensa oil, 1.5 g of Radix bupleuri oil, 0.02 g of vitamin C and 0.02 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 4**

Accurately weigh 25.0 g of Tween-80, 30.0 g of ethanol, and 5.1 g of ethyl acetate, place them into a beaker, manually stir to fully and homogeneously mix the liquid at room temperature of 20 DEG C, directly add 2.0 g of Forsythia suspensa oil, 2.0 g of Radix bupleuri oil, 0.02 g of vitamin C and 0.02 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 5**

Accurately weigh 12.0 g of Tween-80, 16.0 g of ethanol, and 3.0 g of ethyl acetate, place them into a beaker, manually stir to fully and homogeneously mix the liquid at room temperature of 20 DEG C, directly add 0.3 g of Forsythia suspensa oil, 0.3 g of Radix bupleuri oil, 0.02 g of vitamin C and 0.02 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 6**

Accurately weigh 12.0 g of Tween-80, 18.0 g of ethanol and 3.0 g of ethyl acetate, place the mixture into a beaker, manually stir to fully and homogeneously mix the liquid at room temperature of 20 DEG C, directly add 0.5 g of Forsythia suspensa oil, 0.5 g of Radix bupleuri oil, 0.01 g of vitamin C and 0.01 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the product, the total weight thereof is 100.0 g.

**Example 7**

Accurately weigh 24.0 g of Tween-80, 30.0 g of ethanol and 7.0 g of ethyl acetate, place them into a beaker, manually stir to fully and homogeneously mix the liquid at room temperature of 20 DEG C, directly add 3.0 g of Forsythia suspensa oil, 3.0 g of Radix bupleuri oil, 0.01 g of vitamin C and 0.01 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 8**

Accurately weigh 24.0 g of Tween-80, 28.0 g of ethanol and 7.0 g of ethyl acetate, place them into a beaker, manually stir to fully and homogeneously mix the liquid at room temperature of 20 DEG C, directly add 1.0 g of Forsythia suspensa oil, 5.0 g of Radix bupleuri oil, 0.01 g of vitamin C and 0.01 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Example 9**

Accurately weigh 24.0 g of Tween-80, 28.0 g of ethanol and 7.0 g of ethyl acetate, place them into a beaker, manually stir to fully and homogeneously mix the liquid at room temperature of 20 DEG C, directly add 5.0 g of Forsythia suspensa oil, 1.0 g of Radix bupleuri oil, 0.01 g of vitamin C and 0.01 g of vitamin $B_1$, stir thoroughly, and then slowly add distilled water while manually stirring, the system viscosity gradually varies from being small in the very beginning to large with the increasing of amount of the distilled water added, keep dropwise adding while stirring, when the system suddenly becomes thin, the product obtained at this time is the stable faint yellow transparent Forsythia suspensa-Radix bupleuri oil nanoemulsion oral liquid, weigh the liquid, the total weight thereof is 100.0 g.

**Claims**

1. A Forsythia suspensa-Radix bupleuri oil nanoemulsion preparation, **characterized in that**, it is prepared from raw materials with the following percentages by weight: 12.00%-28.00% of Tween-80, 15.00%-30.00% of ethanol, 3.00%-8.00% of ethyl acetate, 0.10%-5.00% of Forsythia suspensa oil, 0.10%-5.00% of Radix bupleuri oil, 0.01 %-0.05% of vitamin C, 0.01 %-0.05% of vitamin B1 and 20.00%-68.40% of distilled water, the total percentages by weight of all the raw materials being 100%; and further **characterized in that** the preparation is for oral use or for use as spray in the air and has antipyretic, antibacterial and virus-inhibiting effects.

2. The Forsythia suspensa-Radix bupleuri oil nanoemulsion preparation for use according to claim 1, **characterized in that**, it is prepared from raw materials with the following percentages by weight: 16.00%-28.00% of Tween-80, 16.00%-30.00% of ethanol, 4.00%-8.00% of ethyl acetate, 1.00%-5.00% of Forsythia suspensa oil, 1.00%-5.00% of Radix bupleuri oil, 0.01 %-0.05% of vitamin C, 0.01 %-0.05% of vitamin B1 and 20.00%-60.00% of distilled water, the total percentages by weight of all the raw materials being 100%.

3. The Forsythia suspensa-Radix bupleuri oil nanoemulsion preparation for use according to claim 1, **characterized in that**, it is prepared from raw materials with the following percentages by weight: 24.00% of Tween-80, 30.00% of ethanol, 7.00% of ethyl acetate, 3.00% of Forsythia suspensa oil, 3.00% of Radix bupleuri oil, 0.01 % of vitamin C, 0.01 % of vitamin B1
and 32.98% of distilled water, the total percentages by weight of all the raw materials being 100%.

**Patentansprüche**

1. Nanoemulsionspräparat aus Öl von Forsythia suspensa und Radix bupleuri, **dadurch gekennzeichnet, dass** es aus Rohstoffen mit den folgenden Gewichtsprozenten hergestellt ist: 12,00 % bis 28,00 % Tween-80, 15,00 % bis 30,00 % Ethanol, 3,00 % bis 8,00 % Ethylacetat, 0,10 % bis 5,00 % Forsythia-suspensa-Öl, 0,10 % bis 5,00 % Radix-bupleuri-Öl, 0,01 % bis 0,05 % Vitamin C, 0,01 % bis 0,05 % Vitamin B1 und 20,00 % bis 68,40 % destilliertem Wasser, wobei die gesamten Gewichtsprozente aller Rohstoffe 100 % betragen; und ferner **dadurch gekennzeichnet, dass** das Präparat zur oralen Verwendung oder zur Verwendung als Spray in der Luft bestimmt ist und antipyretische, antibakterielle und virushemmende Wirkungen aufweist.

2. Nanoemulsionspräparat aus Öl von Forsythia suspensa und Radix bupleuri zur Verwendung nach Anspruch 1, **dadurch gekennzeichnet, dass** es aus Rohstoffen mit den folgenden Gewichtsprozenten hergestellt ist: 16,00 % bis 28,00 % Tween-80, 16,00 % bis 30,00 % Ethanol, 4,00 % bis 8,00 % Ethylacetat, 1,00 % bis 5,00 % Forsythia-suspensa-Öl, 1,00 % bis 5,00 % Radix-bupleuri-Öl, 0,01 % bis 0,05 % Vitamin C, 0,01 % bis 0,05 % Vitamin B1 und 20,00 % bis 60,00 % destilliertem Wasser, wobei die gesamten Gewichtsprozente aller Rohstoffe 100 % betragen.

3. Nanoemulsionspräparat aus Öl von Forsythia suspensa und Radix bupleuri zur Verwendung nach Anspruch 1, **dadurch gekennzeichnet, dass** es aus Rohstoffen mit den folgenden Gewichtsprozenten hergestellt ist: 24,00 % Tween-80, 30,00 % Ethanol, 7,00 % Ethylacetat, 3,00 % Forsythia-suspensa-Öl, 3,00 % Radix-bupleuri-Öl, 0,01 % Vitamin C, 0,01 % Vitamin B1 und 32,98 % destilliertem Wasser, wobei die gesamten Gewichtsprozente aller Rohstoffe 100 % betragen.

**Revendications**

1. Préparation d'une nanoémulsion d'huiles essentielles de Forsythia suspensa-Radix bupleuri, **caractérisée par le fait qu'**elle est préparée à partir de matières de départ avec les pourcentages en poids suivants : 12,00 %-28,00 % de Tween-80, 15,00 %-30,00 % d'éthanol, 3,00 %-8,00 % d'acétate d'éthyle, 0,10 %-5,00 % d'huile essentielle de Forsythia suspensa, 0,10 %-5,00 % d'huile essentielle de Radix bupleuri, 0,01 %-0,05 % de vitamine C, 0,01 %-0,05 % de vitamine B1 et 20,00 %-68,40 % d'eau distillée, les pourcentages totaux en poids de toutes les matières de départ étant de 100 % ; et en outre **caractérisée par le fait que** la préparation est destinée à une utilisation orale ou à une utilisation comme pulvérisation dans l'air et a des effets antipyrétiques, antibactériens et d'inhibition de virus.

2. Préparation d'une nanoémulsion d'huiles essentielles de Forsythia suspensa-Radix bupleuri pour une utilisation selon la revendication 1, **caractérisée par le fait qu'**elle est préparée à partir de matières de départ avec les pourcentages suivants en poids : 16,00 %-28,00 % de Tween-80, 16,00 %-30,00 % d'éthanol, 4,00 %-8,00 % d'acétate d'éthyle, 1,00 %-5,00 % d'huile essentielle de Forsythia suspensa, 1,00 %-5,00 % d'huile essentielle de Radix bupleuri, 0,01 %-0,05 % de vitamine C, 0,01 %-0,05 % de vitamine B1 et 20,00 %-60,00 % d'eau distillée, les pourcentages totaux en poids de toutes les matières de départ étant de 100 %.

3. Préparation d'une nanoémulsion d'huiles essentielles de Forsythia suspensa-Radix bupleuri pour une utilisation selon la revendication 1, **caractérisée par le fait qu'**elle est préparée à partir de matières de départ avec les pourcentages suivants en poids : 24,00 % de Tween-80, 30,00 % d'éthanol, 7,00 % d'acétate d'éthyle, 3,00 % d'huile essentielle de Forsythia suspensa, 3,00 % d'huile essentielle de Radix bupleuri, 0,01 % de vitamine C, 0,01 % de vitamine B1 et 32,98 % d'eau distillée, les pourcentages totaux en poids de toutes les matières de départ étant de 100 %.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Handbook of Chemical Products. Chemical Industry Press